# EUROPEAN PATENT APPLICATION

(11) **EP 3 695 886 A1**
(43) Date of publication of application: **19.08.2020**
(21) Application number: 18866223.3
(22) Date of filing: 10.10.2018
(51) Int. Cl.: A62D 3/38, A01N 59/00, A01P 3/00, A61L 2/20, A62D 101/22, A62D 101/26, A62D 101/28

(54) **METHOD FOR TREATING HARMFUL SUBSTANCE AND OZONE GENERATOR**

(30) Priority: 13.10.2017 JP 2017199714
(71) Applicant: Tamura Teco Co., Ltd., Higashi-Osaka-shi, Osaka 577-0012 (JP); Kinki University, Higashiosaka-shi, Osaka 577-8502 (JP)
(72) Inventor: TAMURA Kozo, Higashi-Osaka-shi Osaka 577-0012 (JP); ISHIWATA Shunji, Higashi-Osaka-shi Osaka 577-8502 (JP); INOUE Tomomi, Higashi-Osaka-shi Osaka 577-8502 (JP); KOTAKE Takeshi, Higashi-Osaka-shi Osaka 577-8502 (JP)
(74) Representative: Lemcke, Brommer & Partner Patentanwälte Partnerschaft mbB
(86) International application number: PCT/JP2018/037679
(87) International publication number: WO 2019/073996

(57) **Abstract**

Provided is a treatment method capable of degrading or sterilizing harmful substances or microorganisms of which the degradation, detoxification, or the like is not easy for ozone gas alone.

A harmful substance treatment method is one that makes ozone gas act on harmful substances, microorganisms, or the like in an environment humidified using a surfactant solution. The harmful substances include anticancer drugs. As a surfactant to be used, a non-ionic surfactant, an anionic surfactant, a cationic surfactant, or an amphoteric surfactant can be used.

## Description

### [Technical Field]

The present invention relates to a technique for degrading or detoxifying (these are referred to as "treating") harmful substances such as harmful compounds and harmful microorganisms by ozone gas.

### [Background Art]

Anticancer drugs are widely used in cancer therapy together with cancer removal surgery and radiotherapy. Anticancer drugs are administered to patients orally or intravenously. It is well known that patients administered anticancer drugs experience adverse drug reaction such as alopecia, nausea, myelosuppression, oral ulceration, and rough skin. This is because anticancer drugs not only act on cancer cells but disrupt even normal cells.

Anticancer drugs cause genetic disorders also in healthy people, and serve as strong carcinogens in terms of blocking cell division. In recent years, a health hazard problem posed by exposure of medical professionals prescribing anticancer drugs, such as doctors and pharmacists, to the anticancer drugs have been made apparent (Non-Patent Literatures 1 to 3).

For this problem, there has been proposed a technique for degrading an anticancer drug using ozone that is widely used for odor removal, degradation of harmful compounds, sterilization, and the like (Patent Literature 1).

### [Citation List]

### [Patent Literatures]

[Patent Literature 1]
Japanese Translation of PCT International Application Publication No. JP-T-2014-208428

### [Non-Patent Literatures]

[Non-Patent Literature 1] Occupational exposure: Risks of healthcare professionals handling anticancer drugs, Med. J. Kinki Univ. Vol.36, No.1, 43-46 2011
[Non-Patent Literature 2] Health Risks for Occupational Exposure to Anticancer (Antineoplastic) Drugs in Health Care Workers, Osaka Prefectural Institute of Public Health, Life and Hygiene Division, Kimiko Tomioka and Shinji Kumagai, Journal of Occupational Health, 2005; 47: 195-203 (Internet, URL: http://joh.sanei.or.jp/pdf/J47/J47_5_01.pdf#search=' %E6%8A%97%E3%81%8C%E3%82%93%ES%89%A4+%E5%8C%BB%E7%99 %82%E5%BE%93%E4%BA%8B%E8%80%85')
[Non-Patent Literature 3] Occupational Exposure to Anticancer Drugs in Health Care Workers, Public Health Laboratories News No.42, published December 24, 2009 (Internet, URL: http://www.iph.pref.osaka.jp/news/vol42/news42.pdf#s earch='%E6%8A%97%E3%81%8C%E3%82%93%E5%89%A4+%E5%8C%B B%E7%99%82%E5%BE%93%E4%BA%8B%E8%80%85')

### [Summary of Invention]

### [Technical Problem]

The technique proposed in Patent Literature 1 is one that enhances the degradation of the anticancer drug by introducing ozone gas into, for example, a safety cabinet and simultaneously providing humidification. The anticancer drug to be degraded includes one scattered into the safety cabinet during preparing an infusion or the like and one attached to medical equipment or the like used for the preparation in the safety cabinet.

However, the technique proposed in Patent Literature 1 is not one that can degrade all of anticancer drugs existing in large numbers. For example, gemcitabine was hard to be degraded or detoxified even by the technique proposed in Patent Literature 1.

The present invention has been made in consideration of the above-described problems, and intends to provide a treatment method capable of treating harmful substances and the like of which the degradation, detoxification, or the like is not easy for ozone gas alone, and an ozone generating device suitable for the treatment.

### [Solution to Problem]

The harmful substance treatment method according to the present invention is one degrading or sterilizing a harmful substance by making ozone gas act in an environment humidified using a solution of a surfactant.

The one other harmful substance treatment method according to the present invention is one degrading or sterilizing a harmful substance by making ozone gas act in a humidified environment in which an object on or through which a surfactant solution or a surfactant is coated or made to permeate is arranged.

Here, the "environment humidified using a solution of a surfactant" means a "space (environment) in which the humidity is increased by vaporizing the solution of the surfactant or other means". The "humidified environment in which an object on or through which a surfactant solution or a surfactant is coated or made to permeate is arranged" means, for example, a "state (environment) where in a space in which the humidity is increased by vaporizing pure water or other means, the object on or through which the surfactant solution or the surfactant is coated or made to permeate is arranged".

Also, the "object" in the "object on or through which a surfactant solution or a surfactant is coated or made to permeate" refers to an object that can hold the surfactant, such as woven fabric, non-woven fabric, glass, ceramic tile, or a metal plate.

As the surfactant used for the harmful substance treatment methods, any of a non-ionic surfactant, an anionic surfactant, a cationic surfactant, and an amphoteric surfactant is selectable.

These harmful substance treatment methods can also be applied to the degradation of an anticancer drug.

The ozone generating device according to the present invention includes: an inflow port for allowing air to flow in; an ozone generator that generates ozone from air flowing in; an outflow port for allowing air containing the generated ozone to flow out; and a degradation enhancing device on or through which a surfactant solution or a surfactant is coated or made to permeate. The degradation enhancing device is arranged in the vicinity of the outflow port so that the air containing the ozone generated by the ozone generator contacts therewith.

As the surfactant coated on or made to permeate through the degradation enhancing device, any of a non-ionic surfactant, an anionic surfactant, a cationic surfactant, and an amphoteric surfactant is selectable.

### [Advantageous Effects of Invention]

According to the present invention, a treatment method capable of degrading harmful substances of which the degradation or the like is not easy for ozone gas alone or enhancing the detoxification of harmful microorganisms, and an ozone generating device used for the treatment can be provided.

### [Brief Description of Drawings]

[Fig. 1] Fig. 1 is a front view of a test system used for an anticancer drug degradation test.
[Fig. 2] Fig. 2 is a plan view of the test system.
[Fig. 3] Fig. 3 shows a calibration curve of gemcitabine by HPLC analysis.
[Fig. 4] Fig. 4 is a chart in which the relationship between a gemcitabine survival rate and A CT value is obtained.
[Fig. 5] Fig. 5 is a chart illustrating the relationship between EO content and the molecular mass of PPO in PEO-PPO copolymer molecules.
[Fig. 6] Fig. 6 is a chart illustrating the relationship between the CT value and a negative rate in Table 1.
[Fig. 7] Fig. 7 is a front view of another test system used for the anticancer drug degradation test.
[Fig. 8] Fig. 8 is a plan view of the test system.
[Fig. 9] Fig. 9 is a front view of a degradation enhancing device.
[Fig. 10] Fig. 10 is a plan view of the degradation enhancing device 19B.

### [Description of Embodiments]

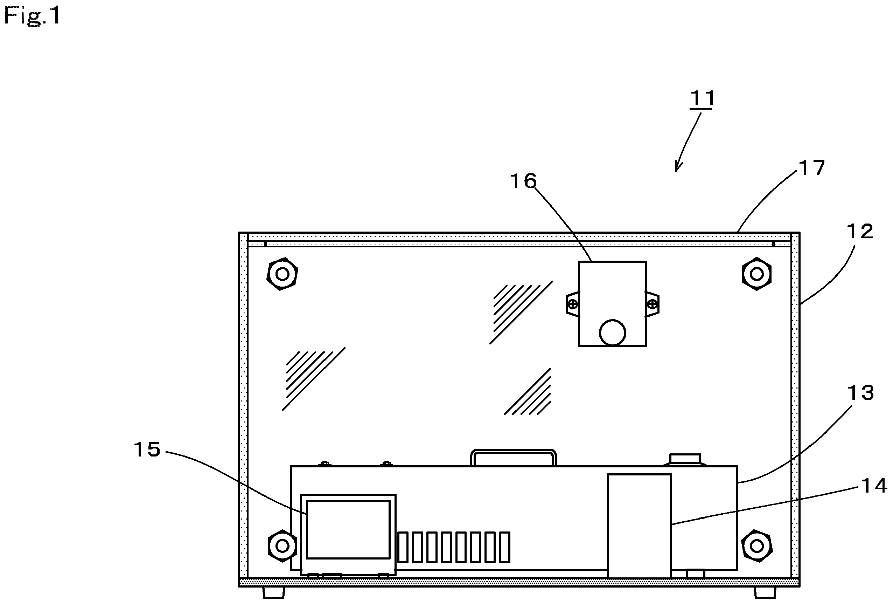

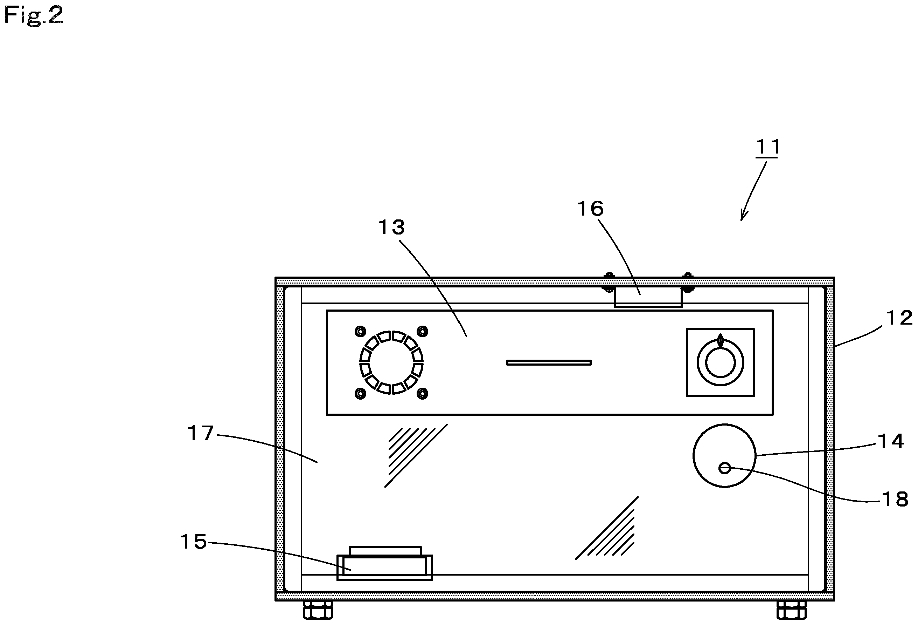

Fig. 1 is a front view of a test system 11 used for an anticancer drug degradation test, and Fig. 2 is a plan view of the test system 11.

The test system 11 includes a container 12, an ozone generator 13, a humidification device 14; a thermo-hygrometer 15, an ozone concentration meter 16, and a CT value recording device.

The container 12 is a rectangular parallelepiped hollow box, whose upper surface is closed by a detachable lid 17.

The container 12 is made of transparent vinyl chloride resin to make it easy to observe the inside from outside.

The ozone generator 13 is a publicly-known stationary ozone gas generating device incorporating an ozone lamp (ultraviolet lamp) and a forced circulation fan.

The humidification device 14 is a device that applies high frequency AC voltage to a piezoelectric vibrator to vibrate it, produces mist from water using the resulting ultrasound waves, and discharges it to the outside from a discharge port 18.

The thermo-hygrometer 15 is a device that measures the temperature and humidity inside the container 12 to display them. The thermo-hygrometer 15 is contained in the container 12.

The ozone concentration meter 16 is publicly-known equipment that is installed in the container 12 to measure ozone gas concentration in the container 12. The ozone concentration meter 16 is externally provided with an unillustrated control part, and the control part digitizes measured ozone concentration to transmit it to the CT value recording device.

The CT value recording device calculates the increment of a CT value (= ozone concentration × interval time) from ozone concentration transmitted from the ozone concentration meter 16 and a transmission interval (time), integrates the increment from a point in time when the degradation treatment was started, and displays an integrated value on a display device thereof. As the CT value recording device, a personal computer is used.

Next, an ozone gas-based gemcitabine degradation test using the test system 11 will be described.

A prepared sample of gemcitabine as a degradation target was obtained by dropping 100 µL of gemcitabine having a concentration of 200 µg/mL onto a square-shaped stainless plate having a side of 100 mm in size and drying it. Hereinafter, a plate attached with gemcitabine after drying is referred to as an "anticancer drug sample".

Gemcitabine used for the anticancer drug sample is Gemzar (registered trademark), a product name, sold by Eli Lilly Japan K.K.

The ozone-based gemcitabine degradation test is conducted with the test system 11 placed in a room in which the temperature is controlled to room temperature (18 to 28 °C). The anticancer drug sample was put in the container 12, and the ozone generator 13 was continuously or intermittently operated until an indication of an integrated CT value of the CT recording device reached a predetermined numerical value. At this time, regarding the humidity inside the container 12, it was noted that the relative humidity was controlled to around 80 % by turning on/off the humidification device 14 while observing humidity displayed on the thermo-hygrometer 15.

The anticancer drug sample taken out of the test system 11 after the ozone-based degradation test, i.e., the attachment on the plate is rinsed into a microtube with 500 µL of Milli-Q water (registered trademark, sold by Merck Millipore K.K.). In the microtube, 18.5 mL of Milli-Q water is preliminarily put. The rinse of the attachment with Milli-Q water into the microtube is conducted three times. The attachment in the microtube was quantitatively analyzed by high-performance liquid chromatography (HPLC). Hereinafter, the solution prepared for the HPLC analysis in this manner is referred to as a "dissolved sample".

Gemcitabine analysis conditions by HPLC are as follows.
Pump: L-2130 manufactured by Hitachi Ltd. (Flow rate 1 mL/min)
Automatic sampler: Model 09 manufactured by System Instruments Co., Ltd. (Injection amount 100 µL)
Detector: SPD-6AV manufactured by Shimadzu Corporation (Wavelength 254 nm)
Column: CAPCELL PAK C18 (registered trademark) manufactured by Shiseido Company, Limited TYPE MG,
SIZE 4.6 mmID × 150 mm
Mobile phase: 50 mmol/L, Phosphoric acid buffer (pH 5.0):Methanol = 85:15

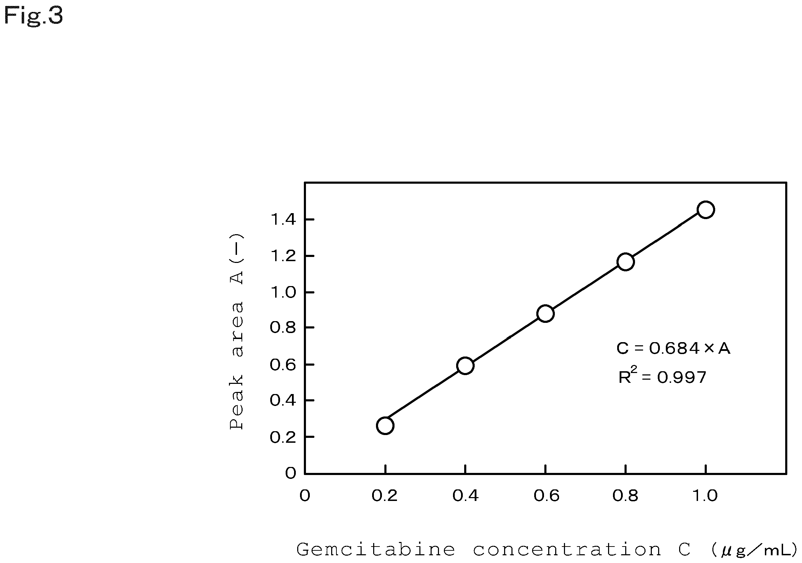

Fig. 3 shows a calibration curve of gemcitabine under the above analysis conditions. The remaining amount of gemcitabine after the degradation test can be obtained by a peak detection result of gemcitabine separated by the high-performance liquid chromatography and the calibration curve.

Table 1 lists the results of measuring the survival rate (undegraded rate) of gemcitabine in the dissolved sample after the degradation test.

Three anticancer samples were prepared for each of Comparative Examples and Examples under the respective conditions, and as the survival rate in the table, the average of the measured values of the three samples was adopted.

**[Table 1]**

| | | Relative humidity (%) | CT value (ppm × min.) | Survival rate (%) | Humidification liquid |
|---|---|---|---|---|---|
| Comparative example | 1 | 80 | 80000 | 100 | Pure water |
| | 2 | 80 | 80000 | 100 | Pure water |
| Example | 1 | 80 | 50000 | 60 | [Chemical 1] Structural formula (Non-ionic |
| | | | | | surfactant) solution |
| | 2 | 80 | 120000 | 0 | [Chemical 1] Structural formula (Non-ionic surfactant) solution |
| | 3 | 80 | 10000 | 75 | L-72 (non-ionic surfactant) 50ppm solution |
| | 4 | 80 | 50000 | 46 | L-72 (non-ionic surfactant) 50ppm solution |
| | 5 | 80 | 10000 | 80 | L-72 (non-ionic surfactant) 500ppm solution |
| | 6 | 80 | 50000 | 56 | L-72 (non-ionic surfactant) 500ppm solution |
| | 7 | 80 | 10000 | 57 | F-68 (non-ionic surfactant) 50ppm solution |
| | 8 | 80 | 50000 | 14 | F-68 (non-ionic surfactant) 50ppm solution |
| | 9 | 80 | 10000 | 46 | F-108 (non-ionic surfactant) 50ppm solution |
| | 10 | 80 | 50000 | 30 | F-108 (non-ionic surfactant) 50ppm solution |
| | 11 | 80 | 10000 | 11 | L-121 (non-ionic surfactant) 50ppm solution |
| | 12 | 80 | 50000 | 1 | L-121 (non-ionic surfactant) 50ppm solution |
| | 13 | 80 | 10000 | 81 | L-31 (non-ionic surfactant) 50ppm solution |
| | 14 | 80 | 50000 | 54 | L-31 (non-ionic surfactant) 50ppm solution |

"Comparative example" indicates a degradation result using only ozone gas under humidification without use of non-ionic surfactant.

Comparative Examples in Table 1 correspond to degradation treatment with only water put in the humidification device 14 for humidification. Examples in Table 1 correspond to degradation treatment with non-ionic surfactant-dissolved water put in the humidification device 14 for humidification.

The details of a non-ionic surfactant used in Examples 1 and 2 are unclear. However, from analysis results of atmospheric pressure chemical ionization (APCI), nuclear magnetic resonance spectroscopy for hydrogen atom (1H NMR), and gel permeation chromatography at Sumika Chemical Analysis Service, Ltd. it was an ethylene oxide-propylene oxide block copolymer (Chemical 1), which has no end alkyl group, whose weight average molecular mass was 2100, and whose number average molecular mass was 1100.

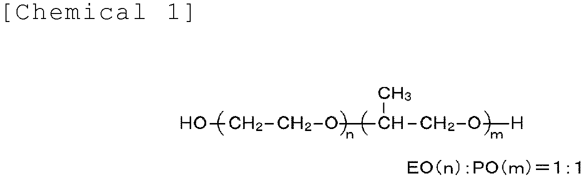

Non-ionic surfactants used in Examples 3 to 14 are product numbers L-72, F-68, F-108, L-121, and L-31 of Adeka Pluronic L · P · F ("Adeka" and "Pluronic" are both registered trademarks) among products manufactured by ADEKA Corporation.

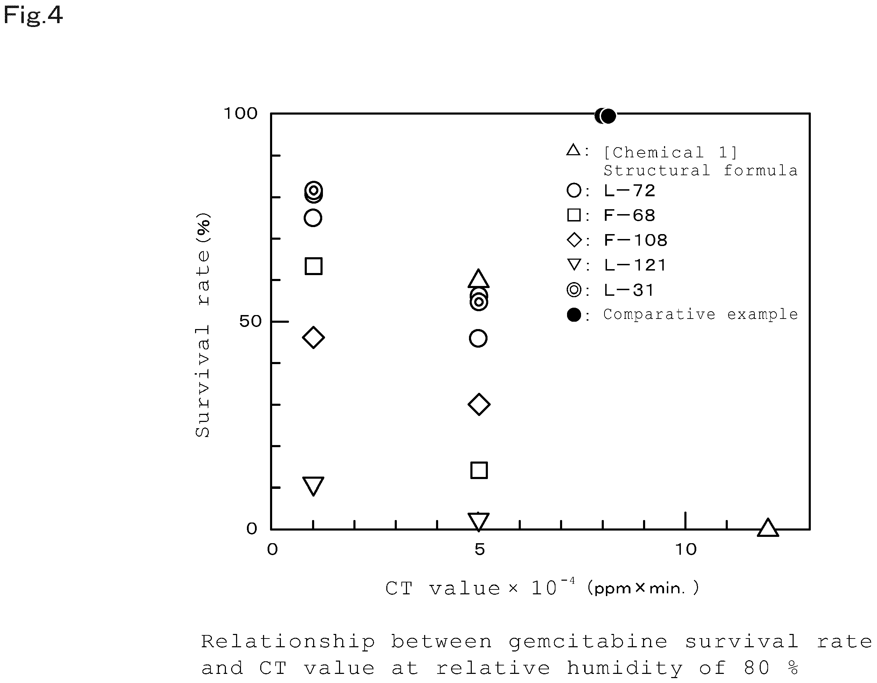

Fig. 4 is a chart in which the relationship between the gemcitabine survival rate and the CT value is obtained from Table 1. It turns out from Fig.4 that in the case of humidification by a non-ionic surfactant solution, as the CT value is increased, gemcitabine is more degraded and its survival rate reduces.

Then, after the gemcitabine degradation treatment was conducted with humidification provided by a non-ionic surfactant solution, the same test system 11 was used to conduct similar treatment with humidification provided by water alone. This is to supplement Comparative Examples (without a non-ionic surfactant) of gemcitabine degradation. However, the results of the ozone-based degradation test in an environment humidified by water alone exhibited that gemcitabine degradation progressed unexpectedly.

Therefore, the inside of the test system 11 and the surfaces of the ozone generator 13 and the like to be contained were cleaned to remove the non-ionic surfactants that probably remained. After that, fabric coated with the non-ionic surfactant L-72 and superior in water absorbency was hung on the lid 17 to provide humidification with only water put in the humidification device 14, and the anticancer drug sample (gemcitabine) degradation treatment was conducted. The resulting results are listed in Table 2.

**[Table 2]**

| | | Relative humidity (%) | CT value (ppm × min.) | Survival rate (%) | Remarks |
|---|---|---|---|---|---|
| Example | 15 | 80 | 50000 | 44 | L-72 (non-ionic surfactant) alone |
| | 16 | 80 | 50000 | 51 | L-72 (non-ionic surfactant) alone |

Table 2 shows that even when without being dissolved in water, placed alone in a humidified environment, the non-ionic surfactant contributes to the degradation of gemcitabine.

The non-ionic surfactant can be expected to produce the same effect even when coated on glass, metal, or the like and placed in a humidified environment, in addition to being used made to permeate through fabric.

Incidentally, from the description in the material "ADEKA, Surfactant product lists (URL: https://www.adeka.co.jp/chemical/products/surface/do wnload/ADEKA KAIMENKASSEIZAI PRODUCTLIST 1012.pdf#se arch%27%EF%BC%A1%EF%BC%A4%EF%BC%A5%EF%BC%AB%EF%BC%A 1+%E3%83%97%E3%83%AB%E3%83%AD%E3%83%8B%E3%83%83%E3%8 2%AF%27)", the non-ionic surfactants L-72, F-68, F-108, L-121, and L-31 in Table 1 and Table 2 are all polyoxyethylene-polyoxypropylene copolymers having no end alkyl group, whose molecular structures are common to the non-ionic surfactant in Examples 1 and 2.

From the above, it can be said that gemcitabine hard to be degraded by ozone gas alone can be degraded by making a non-ionic surfactant coexist under a humidified environment.

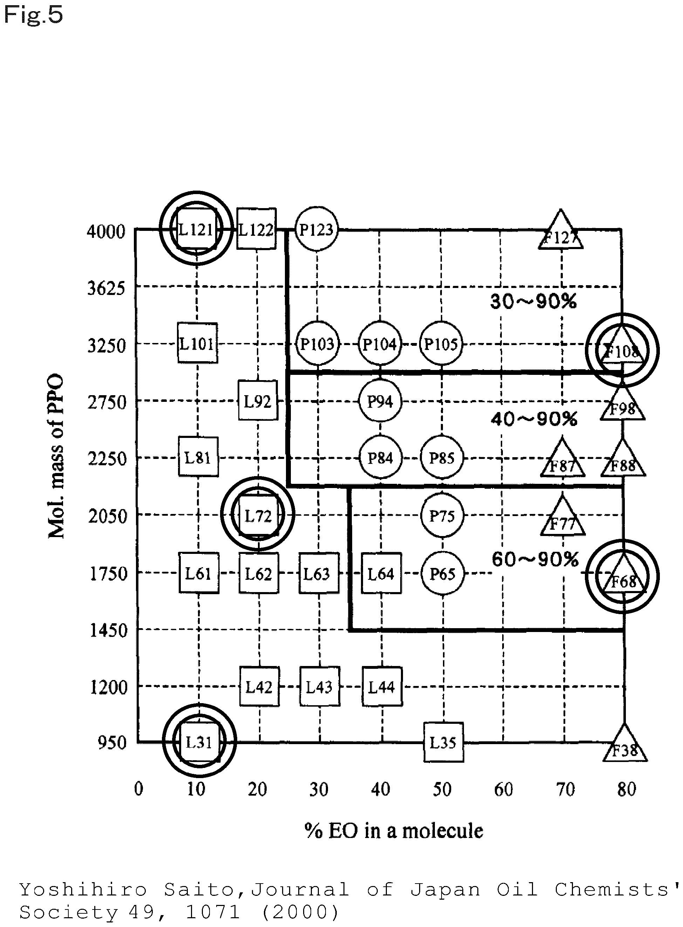

Fig. 5 is a chart illustrating the relationship between oxyethylene (EO) content (%) and the molecular mass of polyoxypropylene (PPO) in molecules of commercially available polyoxyethylene-polyoxypropylene copolymers (Pluronic Grid, Yoshihiro Saito, Journal of Japan Oil Chemists' Society 49, 1071 (2000)).

In Fig. 5, product numbers marked with a double circle represent the non-ionic surfactants used in respective Examples in Table 1. It turns out from Fig. 5 that in the case of polyoxyethylene-polyoxypropylene copolymers, a wide range of combinations of the EO content and the PPO molecular mass enables gemcitabine to be degraded by ozone gas.

Since the coexistence of a non-ionic surfactant enables gemcitabine to be degraded by ozone, it is conceivable that this effect by a non-ionic surfactant is also exerted on other harmful compounds required to be degraded.

As a non-ionic surfactant that performs the enhancement of degradation by ozone, or the like, in addition to the ethylene oxide-propylene oxide block copolymers, other types of non-ionic surfactants, such as an alkyl ether type and an ester type, can be used.

Table 3 lists the results of conducting an ozone gas-based biological indicator (hereinafter referred to as "BI" in some cases) sterilization test under a humidified environment and examining the difference between the presence and absence of a non-ionic surfactant.

**[Table 3]**

| | | Relative humidity (%) | CT value (ppm × min.) | Negative rate (%) | Humidification liquid |
|---|---|---|---|---|---|
| Comparative example | 3 | 80 | 80000 | 0 | Pure water |
| | 4 | 80 | 100000 | 0 | Pure water |
| | 5 | 80 | 160000 | 22 | Pure water |
| Example | 17 | 80 | 30000 | 33 | L-72 (non-ionic surfactant) |
| | | | | | 50ppm solution |
| | 18 | 80 | 40000 | 67 | L-72 (non-ionic surfactant) 50ppm solution |

For the sterilization, the test system 11 illustrated in Fig. 1 and Fig. 2 was used.

As the BI, a stainless disc type (Bacillus atrophaeus 106) manufactured by Mesa Laboratories, Inc. of USA and sold by RAVEN JAPAN CO., LTD. was used. Each sterilization treatment was conducted with nine BIs scattered in the test system 11. BIs after sterilization treatment were incubated at 32.5 ± 2.5 °C for three days, and the presence or absence of coloring, i.e., the presence or absence of remaining bacteria was examined to determine BIs having no remain to be negative. A negative rate in the table represents the percentage of uncolored ones among nine BIs.

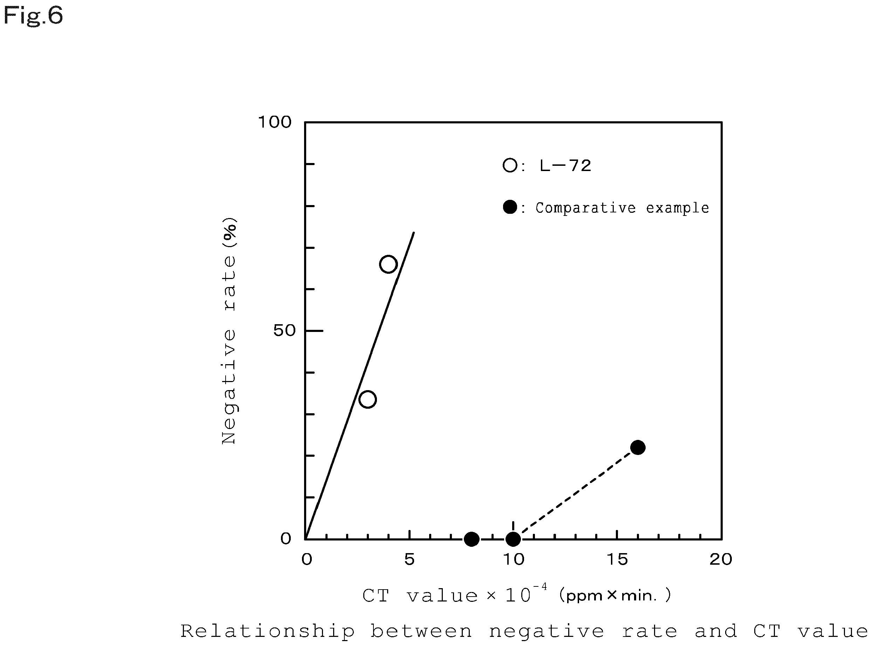

Fig. 6 is a chart illustrating the relationship between the CT value and the negative rate in Table 1.

Fig. 6 shows that Bacillus atrophaeus (Bacillus subtilis) as a sterilization index is remarkable when humidification is provided by the non-ionic surfactant solution as compared with humidification by pure water alone.

Since non-ionic surfactants enable gemcitabine to be degraded by ozone gas, and enhance the bactericidal action as described above, the non-ionic surfactants function to enhance the ozone-based degradation action on other harmful compounds as well.

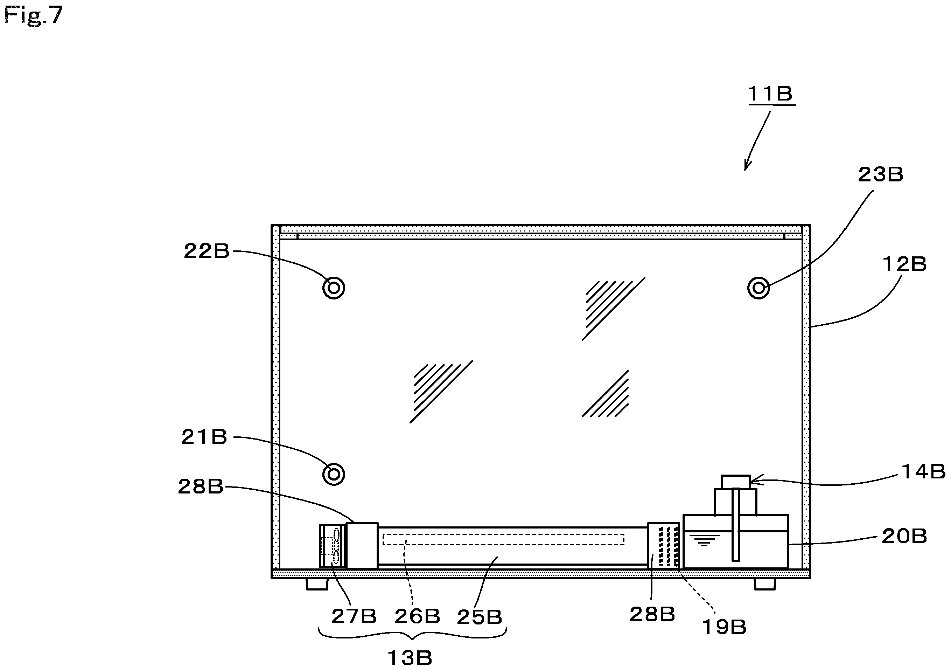

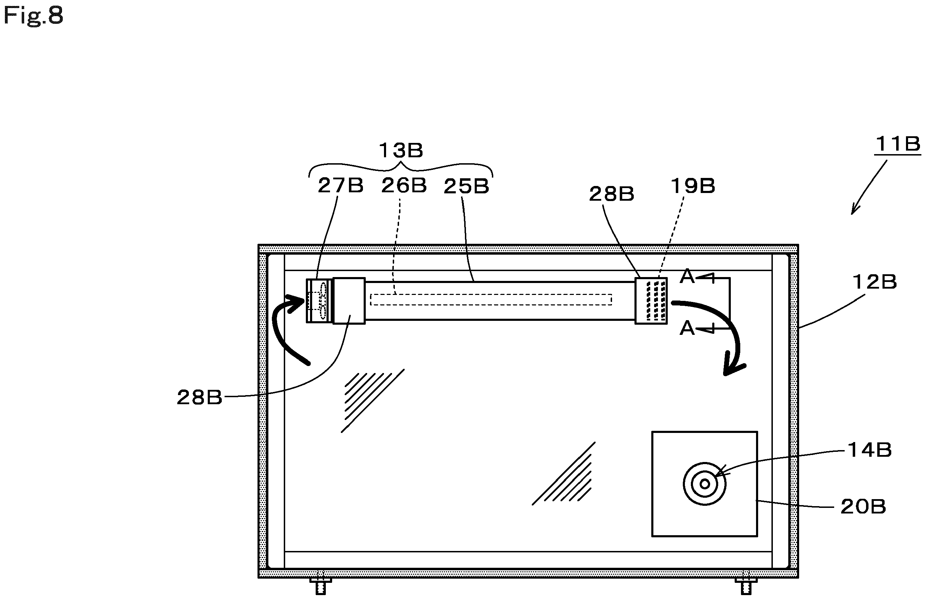

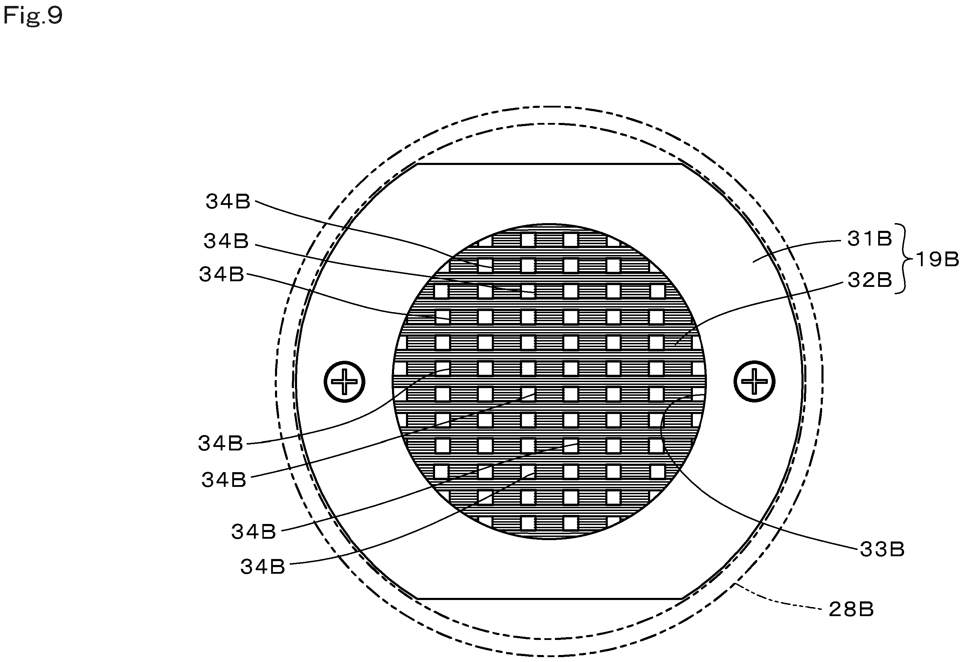

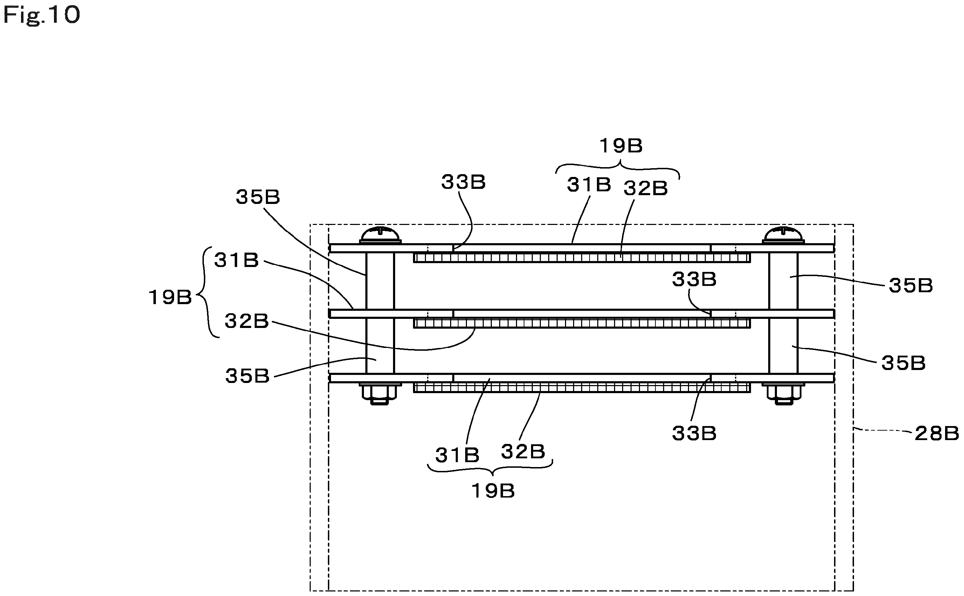

Fig. 7 is a front view of one other test system 11B used for the anticancer drug degradation test, Fig. 8 is a plan view of the test system 11B, Fig. 9 is a front view of a degradation enhancing device 19B, and Fig. 10 is a plan view of the degradation enhancing device 19B. In addition, Fig. 9 is an A-A arrow view in Fig. 8.

The test system 11B includes a container 12B, an ozone generator 13B, the degradation enhancing devices 19B, a humidification device 14B, a thermometer, a hygrometer, an ozone concentration meter, and a CT value recording device.

The container 12B is a rectangular parallelepiped hollow box, and an outline is the same as that of the above-described container 12. The container 12B is provided at the front with two exhaust ports 21B, 22B for humidity measurement and ozone concentration measurement. Further, the front of the container 12B is provided with an intake port 23B for supplementing a gas intake amount to prevent the inside of the container 12B from being excessively depressurized by these types of measurement.

The ozone generator 13B has a tubular body 25B, an ozone lamp (ultraviolet lamp) 26B, and a forced circulation fan 27B.

The tubular body 25B is such that the main body is formed of a circular tube made of vinyl chloride resin and at both ends thereof are connected with short tubes 28B, 28B having a slightly larger diameter than that of the main body and made of vinyl chloride resin. The ozone lamp (ultraviolet lamp) 26B is a publicly-known ozone generating lamp of an elongated cylindrical shape, and contained inside the main body of the tubular body 25B.

The forced circulation fan 27B is fixed to a short tube 28B at one end of the main body with its discharge side facing inward of the main body. As the forced circulation fan 27B, an AC fan (an axial flow fan operable by alternating current) is used.

The degradation enhancing device 19B includes a support frame 31B and a perforated body 32B. The support frame 31B is a substantially circular thick plate having a diameter equal to the inside diameter of the short tubes 28B and made of vinyl chloride resin. The support frame 31B has a perforated hole 33B that is circular concentric with the outer circumference thereof, whose shape is annular.

The perforated body 32B is formed of a plate made of vinyl chloride resin, and both surfaces thereof are provided with fine unevenness. The perforated body 32B has multiple vent holes 34B, ..., 34B penetrating through both surfaces thereof. The perforated body 32B is integrated with the support frame 31B while covering the hole 33B. Accordingly, both sides of the support frame 31B (hole 33B) are communicated by the vent holes 34B.

For the perforated body 32B, another configuration can be adopted. It is, for example, a coarse wire mesh, a metallic porous plate having roughened surfaces, or the like.

The three degradation enhancing devices 19B are integrated interposing spacers 35B in a manner such that the centers of the holes 33B of them are arranged on a straight line at regular intervals, i.e., the respective support frames 31B are parallelized. The three degradation enhancing devices 19B, 19B, 19B are immersed in, for example, a non-ionic surfactant having fluidity (or a solution of it, or the like) before the degradation test, and subjected to drying or the like to the extent that the non-ionic surfactant or the like does not drip.

The degradation enhancing devices 19B, 19B, 19B holding the non-ionic surfactant or the like are contained in the short tube 28B on the side opposite to the forced circulation fan 27B.

The humidification device 14B is one that uses a capillary phenomenon to suck up water inside a container 20B integrated with a vibration device and applies ultrasonic vibration to it to produce mist.

The thermometer (thermocouple) is provided inside the container 12B, and compensating lead wires are connected to a recording device through a side surface of the container 12B.

The hygrometer is arranged outside the container 12B, and connected to the exhaust port 21B for humidity measurement by a tube made of soft Teflon (registered trademark) resin to measure the humidity of gas inside the container 12B sucked by a suction pump.

The ozone concentration meter is arranged outside the container 12B, measures the ozone concentration of the gas inside the container 12B passing through a tube connected to the exhaust port 22B for ozone concentration measurement and made of soft Teflon (registered trademark) resin, and sends the measurement result to the CT value recording device.

The CT value recording device is the same as the CT value recording device in the above-described test system 11.

The gemcitabine degradation test by the one other test system 11B was conducted using one prepared in the same manner as that for the prepared sample of gemcitabine (anticancer drug sample) in the above-described test system 11.

In the degradation test, the anticancer drug sample is put in the container 12B of the test system 11B installed in a room in which the temperature is controlled to room temperature (18 to 28 °C).

The degradation of gemcitabine was conducted until an indication of an integrated CT value of the CT recording device reached a predetermined numerical value while operating the ozone generator 13B. The humidification device 14B was turned on/off so that the relative humidity inside the container 12B was controlled to around 80 %.

Post-treatment, remaining amount analysis, and the like of the anticancer drug sample taken out of the test system 11B after the ozone-based degradation test were conducted by the same methods as the methods in the degradation test using the test system 11.

Table 4 lists the results of measuring the survival rate (undegraded rate) of gemcitabine after the degradation test when humidification was provided with a surfactant solution put in the container 20B of the humidification device 14B. Surfactants put in the container 20B of the humidification device 14B in respective Examples are listed in the "humidification liquid" column of Table 4. In these degradation tests, the degradation enhancing device 19 is not used.

Table 5 lists the results of conducting the gemcitabine degradation test with the perforated bodies 32B of the degradation enhancing devices 19B made to hold a surfactant and contained in the short tube 28B on the side opposite to the forced circulation fan 27B. In this case, pure water is put in the container 20B of the humidification device 14B. Surfactants used in respective Examples are listed in the "surfactant coated on degradation enhancing devices" column.

In both Table 4 and Table 5, during the degradation test, the forced circulation fan 27B was operated to produce a gas flow in the container 12B,

**[Table 4]**

| | | Relative humidity (%) | CT value (ppm × min.) | Survival rate (%) | Humidification liquid |
|---|---|---|---|---|---|
| Example | 19 | 80% | 10000 | 66 | F-108 50ppm solution |
| | 20 | 80% | 50000 | 0 | F-108 50ppm solution |
| | 21 | 80% | 50000 | 62 | LA-875 50ppm solution |
| | 22 | 80% | 50000 | 69 | LA-93 50ppm solution |
| | 23 | 80% | 10000 | 79 | Sorbitan monolaurate 50ppm solution |
| | 24 | 80% | 50000 | 26 | Sorbitan monolaurate 50ppm solution |
| | 25 | 80% | 50000 | 59 | Polyethylene glycol monolaurate 50ppm solution |
| | 26 | 80% | 10000 | 71 | Sodium lauryl sulfate (anionic surfactant) 50ppm solution |
| | 27 | 80% | 50000 | 21 | Sodium lauryl sulfate (anionic surfactant) 50ppm solution |
| | 28 | 80% | 10000 | 70 | Lauryltrimethylammonium chloride (cationic surfactant) 50ppm solution |
| | 29 | 80% | 50000 | 53 | Lauryltrimethylammonium chloride (cationic surfactant) 50ppm solution |
| | 30 | 80% | 10000 | 81 | Lauryldimethylaminoacetic acid (amphoteric surfactant) 50ppm solution |
| | 31 | 80% | 50000 | 76 | Lauryldimethylaminoacetic acid (amphoteric surfactant) 50ppm solution |

**[Table 5]**

| | | Relative humidity (%) | CT value (ppm × min.) | Survival rate (%) | Surfactant coated on degradation enhancing device |
|---|---|---|---|---|---|
| Example | 32 | 80% | 10000 | 12 | L-121 |
| | 33 | 80% | 50000 | 1 | L-121 |
| | 34 | 80% | 10000 | 81 | L-31 |
| | 35 | 80% | 50000 | 54 | L-31 |
| | 36 | 80% | 10000 | 57 | L-68 |
| | 37 | 80% | 50000 | 14 | L-68 |
| | 38 | 80% | 10000 | 46 | F-108 |
| | 39 | 80% | 50000 | 30 | F-108 |
| | 40 | 80% | 50000 | 3 | LA-875 |
| | 41 | 80% | 10000 | 77 | LB-93 |
| | 42 | 80% | 50000 | 31 | LB-93 |
| | 43 | 80% | 10000 | 79 | LB-720 |
| | 44 | 80% | 50000 | 77 | LB-720 |
| | 45 | 80% | 50000 | 68 | Sorbitan monolaurate |
| | 46 | 80% | 10000 | 36 | Polyoxyethylene sorbitan monolaurate |
| | 47 | 80% | 50000 | 17 | Polyoxyethylene sorbitan monolaurate |
| | 48 | 80% | 50000 | 59 | Polyethylene glycol monolaurate |
| | 49 | 80% | 10000 | 81 | Sodium lauryl sulfate (anionic surfactant) |
| | 50 | 80% | 50000 | 63 | Sodium lauryl sulfate (anionic surfactant) |
| | 51 | 80% | 10000 | 90 | Lauryltrimethylammonium chloride (cationic surfactant) |
| | 52 | 80% | 50000 | 80 | Lauryltrimethylammonium chloride (cationic surfactant) |
| | 53 | 80% | 10000 | 79 | Lauryldimethylaminoacetic acid (amphoteric surfactant) |
| | 54 | 80% | 50000 | 76 | Lauryldimethylaminoacetic acid (amphoteric surfactant) |

Non-ionic surfactants used in Examples 21, 22, and 40 to 44 in Tables 4, 5 are product numbers LA-875, LB-93, and LB-720 of ADEKA TOL LA · OA ("ADEKA" and "ADEKA TOL" are both registered trademarks) and ADEKA TOLL B among products manufactured by ADEKA CORPORATION.

Sorbitan monolaurate (non-ionic surfactant) used in Examples 23, 24, and 45 is a reagent sold by FUJIFILM Wako Pure Chemical Corporation.

Polyethylene glycol monolaurate (non-ionic surfactant) used in Examples 25 and 48 is a reagent sold by FUJIFILM Wako Pure Chemical Corporation.

Sodium lauryl sulfate (anionic surfactant) used in Examples 26, 27, 49, and 50 is a reagent sold by FUJIFILM Wako Pure Chemical Corporation.

Lauryltrimethylammonium chloride (cationic surfactant) used in Examples 28, 29, 51, and 52 is a reagent sold by FUJIFILM Wako Pure Chemical Corporation.

Lauryldimethylaminoacetic acid (amphoteric surfactant) used in Examples 30, 31, 53, and 54 is a reagent sold by FUJIFILM Wako Pure Chemical Corporation.

Polyethylene sorbitan monolaurate (non-ionic surfactant) used in Examples 46 and 47 is a reagent sold by FUJIFILM Wako Pure Chemical Corporation.

From Table 4, in the ozone-based gemcitabine degradation conducted with humidification provided using a surfactant solution, the alkyl ether type non-ionic surfactants (Examples 21 and 22) and the ester type non-ionic surfactants (Examples 23, 24, and 25) have an effect of being able to degrade gemcitabine as with the ethylene oxide-propylene oxide block copolymer type (Table 1 and Examples 19 and 20).

Further, it turns out from Table 4 that even under an environment humidified using the anionic surfactant, the cationic surfactant, or the amphoteric surfactant, gemcitabine can be degraded by ozone (Examples 26 to 31).

The respective results listed in Table 5 are ones of reconfirming the degradation test results listed in Table 2 and examining whether application to a surfactant other than a non-ionic surfactant is possible or not. In each Example in Table 5, a result was obtained in a manner such that humidification was provided using pure water, a surfactant (the degradation enhancing devices 19B) was arranged in the container 12B of the test system 11B, and gemcitabine was degraded by ozone.

It turns out from the results in Table 5 that not a method that mixes a surfactant with humidification water, but even a method that arranges a surfactant alone in a degradation environment (the container 12B of the test system 11B) allows the surfactant to significantly contribute to improving the degradation power of ozone. Also, even when using the anionic surfactant (Examples 49 and 50), the cationic surfactant (Example 52), or the amphoteric surfactant (Examples 53 and 54) as a surfactant in addition to the ethylene oxide-propylene oxide block copolymer type (Examples 32 to 39), alkyl ether type (Examples 40 to 44), or ester type (Example 45 to 48) non-ionic surfactant, gemcitabine can be degraded by ozone.

In the ozone-based degradation treatment using the degradation enhancing devices 19B, ozone generated by the ozone generator 13B passes through the vent holes 34B provided in the outlet of the tubular body 25B, and at this time, contacts with a surfactant coated on the perforated bodies 32B. If the surfactant enhances the action of ozone, the test system 11B in Figs. 7 and 8 in which the surfactant is arranged on the discharge side (outflow port) of the ozone generator 13B more efficiently activates ozone as compared with a method that arranges a surfactant in another place to allow ozone floating in an environment to contact with it.

The ozone generator 13B and the degradation enhancing devices 19B can be considered as an ozone generating device. The ozone generating device can be said to be such that the tubular body 25B in which the ozone lamp 26B is arranged has an inflow port (e.g., an opening in a connection part to a short tube 28B) for allowing air in a degradation environment to flow in and an outflow port (e.g., an opening in a connection part to the other short tube 28B) for allowing air containing generated ozone to flow out into the degradation environment. As a result, the degradation enhancing devices 19B are arranged in the vicinity of the outflow port so that the air containing the generated ozone efficiently contacts therewith, and can more effectively activate ozone.

In the ozone generating device, even when fabric or the like on or through which a surfactant solution or a surfactant is coated or made to permeate is arranged just near the outflow port in place of the perforated bodies 32B of the degradation enhancing devices 19B, the effect of enhancing the degradation capability of ozone can be expected.

In the test system 11B, the discharge side of the forced circulation fan 27B is directly connected to the inflow port of the above ozone generating device. However, as long as it is possible to efficiently send air to the inflow port, gas flow producing means (gas stirring means) in the degradation environment is not required to be directly connected to the inflow port of the ozone generating device, and a device other than the forced circulation fan 27B can replace the function of it.

In the above-described embodiment, gemcitabine is mainly used as a harmful compound degradation target. However, the above-described method is effective for enhancing the degradation treatment using ozone gas, and without limitation to the degradation of gemcitabine, can be used to degrade other harmful drugs and harmful compounds that are hard to be degraded by ozone gas alone. Also, as shown in Table 3 and Fig. 6, the above-described method can be used to detoxify microorganisms such as bacteria.

Besides, the test systems 11, 11B and the respective configurations or overall structures, shapes, dimensions, numbers, materials, or the like of the test systems 11, 11B can be appropriately changed in accordance with the scope of the present invention.

### [Industrial Applicability]

The present invention can be used for the degradation of harmful compounds and the like and the detoxification of harmful microorganisms, which are hard for ozone gas alone.

## Claims

1. A harmful substance treatment method degrading or sterilizing a harmful substance by making ozone gas act in an environment humidified using a solution of a surfactant.

2. A harmful substance treatment method degrading or sterilizing a harmful compound by making ozone gas act in a humidified environment in which an object on or through which a surfactant solution or a surfactant is coated or made to permeate is arranged.

3. The harmful substance treatment method according to claim 1 or claim 2, wherein
the surfactant is a non-ionic surfactant.

4. The harmful substance treatment method according to claim 1 or claim 2, wherein
the surfactant is an anionic surfactant.

5. The harmful substance treatment method according to claim 1 or claim 2, wherein
the surfactant is a cationic surfactant.

6. The harmful substance treatment method according to claim 1 or claim 2, wherein
the surfactant is an amphoteric surfactant.

7. The harmful substance treatment method according to any one of claims 1 to 6, wherein
the harmful substance is an anticancer drug.

8. The harmful substance treatment method according to claim 3, wherein
the surfactant is an ethylene oxide-propylene oxide block copolymer.

9. An ozone generating device comprising:
an inflow port for allowing air to flow in;
an ozone generator that generates ozone from air flowing in;
an outflow port for allowing air containing the generated ozone to flow out; and
a degradation enhancing device on or through which a surfactant solution or a surfactant is coated or made to permeate, wherein
the degradation enhancing device is arranged in a vicinity of the outflow port so that the air containing the generated ozone contacts therewith.

10. The ozone generating device according to claim 9, wherein
the surfactant is a non-ionic surfactant.

11. The ozone generating device according to claim 9, wherein
the surfactant is an anionic surfactant.

12. The ozone generating device according to claim 9, wherein
the surfactant is a cationic surfactant.

13. The ozone generating device according to claim 9, wherein
the surfactant is an amphoteric surfactant.
